# EUROPEAN PATENT SPECIFICATION

(11) **EP 3 129 499 B1**
(45) Date of publication and mention of the grant of the patent: **01.08.2018**
(21) Application number: 15727065.3
(22) Date of filing: 09.04.2015
(51) Int. Cl.: C12Q 1/68

(54) **UNIVERSAL CONTROLS FOR SEQUENCING ASSAYS**
UNIVERSELLE KONTROLLEN FÜR SEQUENZIERUNGSTESTS
TÉMOINS UNIVERSELS POUR ANALYSE DE SÉQUENÇAGE

(30) Priority: 10.04.2014 GB 201406485
(43) Date of publication of application: 15.02.2017
(73) Proprietor: Vela Operations Singapore Pte. Ltd., 117406 Singapore (SG)
(72) Inventor: LEE, Charlie Wah Heng, Singapore 152075 (SG); ARIYARATNE, Pramila Nuwantha, Singapore 768427 (SG)
(74) Representative: Behrens, Lüder
(86) International application number: PCT/IB2015/052578
(87) International publication number: WO 2015/155723

(56) References cited:
- EP-A1- 2 465 945
- WO-A2-2007/017699
- US-A1- 2001 006 800
- US-A1- 2007 141 563
- TELENTI A ET AL: "Competitive polymerase chain reaction using an internal standard: Application to the quantitation of viral DNA", JOURNAL OF VIROLOGICAL METHODS, ELSEVIER BV, NL, vol. 39, no. 3, 1 September 1992 (1992-09-01), pages 259-268, XP023696621, ISSN: 0166-0934, DOI: 10.1016/0166-0934(92)90099-Y [retrieved on 1992-09-01]
- GILLILAND G: "ANALYSIS OF CYTOKINE MRNA AND DNA: DETECTION AND QUANTITATION BY COMPETITIVE POLYMERASE CHAIN REACTION", PROCEEDINGS OF THE NATIONAL ACADEMY OF SCIENCES, NATIONAL ACADEMY OF SCIENCES, US, vol. 87, no. 7, 1 April 1990 (1990-04-01), pages 2725-2729, XP000368689, ISSN: 0027-8424, DOI: 10.1073/PNAS.87.7.2725

## Description

### FIELD OF THE INVENTION

The present invention relates to universal controls for sequencing assays, which can serve as a positive and negative control and as extraction control, respectively. The present application describes corresponding plasmids, kits, their uses and a method of detecting a specific nucleic acid using the controls of the present invention.

### BACKGROUND OF THE INVENTION

The use of nucleic acid sequencing has become an essential tool in many diagnostic areas in modern medicine. An example of such an area is the oncology, where nucleic acid sequencing is employed in order to identify whether e.g. oncogenic mutations are present in a gene or whether cancer-inducing and/or indicating translocations are present in a genome. Further, nucleic acid sequencing is employed to detect whether a pathogenic microorganism (such as e.g. a bacteria or a virus) is present in a clinical sample, such as e.g. a tissue sample or a blood sample from a human patient. In the latter method, nucleic acid sequences are detected, which are not found in a human subject but only in the microorganism.

Typically, the actual sequencing step is preceded by an amplification reaction in order to amplify the nucleic acid to be sequenced. Such an amplification reaction is usually carried out by a PCR reaction; thus, specific primers are used in the PCR reaction, which hybridize to sequences upstream and downstream of the sequence to be amplified (i.e. the primers flank the sequence to be amplified).

Due to progress over the last decades, many steps during diagnostic methods have been automated. An example of such a step is the extraction of nucleic acids from a clinical sample. Thus, it is now possible to provide a clinical sample more or less in a state "as taken" from a patient and to carry out *inter alia* the extraction step in a fully automated manner. EP2465945 discloses the use of control plasmids in amplification reactions. Given the above, a typical diagnostic assay may be directed to answering the question whether a specific pathogen is present in a clinical sample from a human subject, e.g. a sample derived from the respiratory tract. To this aim, a clinical sample is taken from the human subject (such as e.g. sputum), wherein this step is carried out under as sterile conditions as possible. The sample is then transferred to a reaction vial, which is typically part of a multi-vial system, such as e.g. a 36-vial sample ring. Of course, further clinical samples from other patients may also be analyzed in parallel. The nucleic acids from these samples are then *inter alia* extracted in an automated manner. In the next automated step, PCR reactions are carried out in the vials, wherein specific primers are used, resulting in the amplification of a target sequence, which is only found in the pathogen and characteristic for the specific pathogen to be detected. In the next step, the amplified nucleic acid is then sequenced in order to identify the target sequence.

The assay can either result therein that the target sequence is identified, which would indicate the presence of the pathogen in the clinical sample, or that the target sequence is not detected, which would indicate the absence of the pathogen in the clinical sample.

It can, however, not be excluded in an assay as described above that certain steps of the assay have been conducted improperly.

A typical example of an improperly conducted assay is that the target sequence is not detected because the extraction step has not been carried out properly. Thus, the pathogen might indeed be present in the sample although the result is negative (a "false-negative" result). In order to exclude an improper extraction step, an extraction control is typically carried out; the extraction control usually corresponds to a nucleic acid with a sequence that differs from the sequence to be detected. This nucleic acid is typically added ("spiked") to the sample prior to the beginning of the automated extraction step (usually via adding the nucleic acid to the lysis buffer used during the extraction). Since the sequence of this nucleic acid differs from the sequence to be detected, a second pair of primers is used during the amplification reaction in order to amplify the sequence of the extraction control. The presence of the sequence of the extraction control after completion of the assay indicates that the extraction step has indeed been carried out properly.

An additional pair of primers is thus needed for the extraction control bearing the risk of cross-reactivity with the primers used for target amplification and increasing the costs.

It has further to be ensured that the PCR reaction to amplify the target sequence works under the conditions applied. To this aim, a positive control is applied; the positive control usually consists of a plasmid carrying exactly the viral sequence to be amplified including regions flanking this sequence, which are identical to the sequences, to which the primers used for the sample amplification reactions hybridize. If the sequence can be detected in this positive control using the reagents and conditions as used for the samples, the amplification reaction indeed worked properly. In order to ensure that there was, however, no contamination of all samples with the positive control or the pathogen, a negative control needs to be carried out.

A negative control reaction is typically carried out in a separate vial, wherein only buffer is added to this specific vial instead of a sample. The presence of the target sequence in the negative control indicates that the samples have been contaminated.

An additional control reaction requiring material and space thus needs to be carried out as negative control.

Summarizing the above, the controls as presently used in a detection assay require additional space and material and increase the possibility of cross-reactivity. Thus, there is the need to improve the experimental controls used in diagnostic sequencing assays.

### OBJECTS AND SUMMARY OF THE INVENTION

Accordingly, the present invention provides a new method of detecting the presence of a nucleic acid comprising a target sequence in a sample, a plasmid, the use of said plasmid and a kit for the detection of a nucleic acid comprising a target sequence in a sample.

In a first aspect, the present invention relates to an *in vitro* method of detecting the presence of a nucleic acid comprising a target sequence (T) in a sample, wherein said method comprises the following steps:
a) Providing a sample potentially comprising a nucleic acid comprising T, wherein T is flanked by a sequence hybridizing to a forward primer (FOR) and a sequence hybridizing to a reverse primer (REV);
b) Transferring said sample into a vial V1;
c) Providing a plasmid comprising a control sequence 1 (S1), wherein S1 is flanked by a sequence hybridizing to FOR and a sequence hybridizing to REV, wherein T and S1 are not identical;
d) Transferring said plasmid of step c) into a vial V2;
e) Providing a plasmid comprising a control sequence 2 (S2), wherein S2 is flanked by a sequence hybridizing to FOR and a sequence hybridizing to REV, wherein T, S1 and S2 are not identical;
f) Transferring said plasmid of step e) into said vials;
g) Extracting nucleic acids in said vials;
h) Conducting PCR reactions in said vials using the primers FOR and REV;
i) Sequencing the nucleic acids amplified in said vials;
wherein the presence of T in vial V1 indicates the presence of said nucleic acid comprising T in said sample if the sequencing in vial V2 resulted in the presence of S1 and S2, and if the sequencing in vial V1 resulted in the presence of T and S2.

It needs to be understood that the above results of the sequencing are meant in an exclusive manner, i.e. that no further sequences are identified; accordingly, the passage above may also be formulated as follows: wherein the presence of T in vial V1 indicates the presence of said nucleic acid comprising T in said sample if the sequencing in vial V2 resulted only in the presence of two sequences, namely S1 and S2, and if the sequencing in vial V1 resulted only in the presence of two sequences, namely T and S2. If several target sequences in a nucleic acid are detected, then the sequencing in vial V1 should result in the presence of these several target sequences and S2.

In a preferred embodiment, said sample is a clinical sample, wherein said clinical sample is preferably from a human subject. It can be preferred that said clinical sample is a tissue sample or a body fluid sample. Said sample may e.g. be a tissue sample gained from the respiratory tract, the gastrointestinal tract or from a transplant after transplantation. Further, said sample may in particular be a body fluid sample selected from the group consisting of blood, plasma, serum, lymphatic fluid and saliva.

In another preferred embodiment, said nucleic acid comprising T is a nucleic acid from a microorganism. Preferably, said microorganism is selected from the group consisting of bacteria, archaea, protozoa, fungi and viruses. In a particularly preferred embodiment, said microorganism is a bacterium selected from the group consisting of Group A Streptococcus, *Mycobacterium tuberculosis* Complex members including *Mycobacterium tuberculosis, Mycobacterium africanum, Mycobacterium bovis, Mycobacterium canetti* and *Mycobacterium microti, Salmonella enterica* spp., *Clostridium difficile,* Vancomycin-resistant enterococcus and Methicillin-resistant *Staphylococcus aureus.* In another particularly preferred embodiment, said microorganism is a virus selected from the group consisting of adenovirus, avian influenza A (H7N9) virus, Middle East Respiratory Syndrome Coronavirus, norovirus, BK virus, cytomegalovirus, Epstein-Barr virus, herpex simplex virus 1, herpex simplex virus 2, Varicella-Zoster virus, enterovirus, human immunodeficiency virus (HIV), in particular HIV-1, hepatitis B virus (HBV), hepatitis C virus (HCV), Dengue virus and Chikungunya virus. In the most preferred embodiment, said microorganism is a virus selected from the group consisting of HIV (in particular HIV-1), HBV and HCV.
Within a specific species, different genotypes of a microorganism may be selected and detected, such as e.g. the GI and GII genotypes of a norovirus; the subtypes A to K of HIV; the genotypes A to H of HBV and the genotypes 1 to 4 of HCV.

In a particularly preferred embodiment, said nucleic acid comprising a target sequence (T) is double stranded DNA.

In a particularly preferred embodiment, the method of the present invention may thus be used to detect the presence of a microorganism in a sample, particularly the microorganisms as set out above.

In yet another preferred embodiment, said nucleic acid comprising T is an oncogene, preferably a human oncogene. Preferably, said human oncogene is selected from the group consisting of BCR-ABL major, BCR-ABL minor, BCR-AML1 ETO, PML-RARA, BRAF V600 mutants, KRAS mutants and NRAS mutants.

In a particularly preferred embodiment, the method of the present invention may thus be used to detect the presence of an oncogene in a sample, particularly the oncogenes as set out above.

In still another preferred embodiment, S1 and S2 have no homology to T and are selected such that they are amplified using the reagents and conditions for amplification of T (this applies e.g. to the G/C-content of S1 and S2). S1 and S2 may e.g. be derived from the genome of a plant, in particular of the tobacco mosaic virus (TMV). However, S1 and S2 may also be artificial sequences, which are not naturally occurring.

It can be particularly preferred that T, S1 and S2 have a length of less than or equal to about 2000 bases. Particularly preferred is a length of less than or equal to about 1000 bases, preferably of about 600 bases or about 400 bases.

In another embodiment relating to the first aspect, said plasmids of steps c) and e) are prokaryotic or eukaryotic plasmids. It can be particularly preferred to use a prokaryotic plasmid, in particular a commonly used *E. coli* plasmid.

In another preferred embodiment, said plasmid of step e) is transferred into said vials by adding said plasmid into the lysis buffer, which is used during the extraction of nucleic acids in all vials, and then adding said lysis buffer comprising said plasmid for the extraction step.

In yet another preferred embodiment, said PCR-reactions conducted in step h) are RT-PCR-reactions. This of course particularly applies if the nucleic acid comprising a target sequence (T) is a single stranded or double stranded RNA.

In another preferred embodiment, said extraction step g), said PCR reaction of step h) and said sequencing of step i) are conducted in an optionally fully automated manner. It can be particularly preferred that the following devices are used: a Sentosa SX101 device (Vela Diagnostics) or an epMotion System (Eppendorf) for step g), a Rotor-Gene Q (Qiagen) device in step h) and an Ion Torrent Semiconductor Sequencing device (life technologies) in step i).

In still another preferred embodiment, said sequencing of step i) is conducted by single-molecule real-time sequencing, ion semiconductor sequencing, pyrosequencing, sequencing by synthesis, sequencing by ligation and chain termination sequencing. Particularly preferred is ion semiconductor sequencing.

In yet another preferred embodiment of the first aspect of the invention, the presence of a nucleic acid comprising a target sequence (T) is detected in at least two samples in parallel. Thus, the present invention also relates to an *in vitro* method of detecting the presence of a nucleic acid comprising a target sequence (T) in at least two samples SA1 and SA2, wherein said method comprises the following steps:
a) Providing samples SA1 and SA2 potentially comprising a nucleic acid comprising T, wherein T is flanked by a sequence hybridizing to a forward primer (FOR) and a sequence hybridizing to a reverse primer (REV);
b) Transferring SA1 into a vial V1 and SA2 into a vial V3;
c) Providing a plasmid comprising a control sequence 1 (S1), wherein S1 is flanked by a sequence hybridizing to FOR and a sequence hybridizing to REV, wherein T and S1 are not identical;
d) Transferring said plasmid of step c) into a vial V2;
e) Providing a plasmid comprising a control sequence 2 (S2), wherein S2 is flanked by a sequence hybridizing to FOR and a sequence hybridizing to REV, wherein T, S1 and S2 are not identical;
f) Transferring said plasmid of step e) into said vials;
g) Extracting nucleic acids in said vials;
h) Conducting PCR reactions in said vials using the primers FOR and REV;
i) Sequencing the nucleic acids amplified in said vials;
wherein the presence of T in vials V1 and V3 indicates the presence of said nucleic acid comprising T in samples SA1 and SA2 if the sequencing in vial V2 resulted in the presence of S1 and S2, and if the sequencing in vials V1 and V3 resulted in the presence of T and S2.

The setup described above may thus be used to analyze samples in parallel, preferably up to 15 samples (wherein the analysis of 7 or 15 samples in parallel is particularly preferred); this may also be referred to as multiplex method. It can be preferred to carry out the analysis of 7 samples in parallel (plus an additional control sample 8) if the nucleic acid comprising T is an oncogene; if the nucleic acid comprising T is a nucleic acid from a microorganism, it can be particularly preferred to carry out the analysis of 15 samples in parallel (plus an additional control sample 16). In general, a nucleic acid comprising T is present in a sample if the sequencing in vial V2 resulted in the presence of S1 and S2, and if the sequencing in the corresponding sample vial resulted in the presence of T and S2.

It needs to be understood that in some embodiments of the present invention at least two target sequences comprised in the nucleic acid are analyzed. Thus, the present invention also relates to an *in vitro* method of detecting the presence of a nucleic acid comprising at least two target sequences (T1 and T2) in a sample, wherein said method comprises the following steps:
a) Providing a sample potentially comprising a nucleic acid comprising T1 and T2, wherein T1 is flanked by a sequence hybridizing to a forward primer (FOR) and a sequence hybridizing to a reverse primer (REV) and wherein T2 is flanked by a sequence hybridizing to a forward primer (FOR1) and a sequence hybridizing to a reverse primer (REV1); wherein T1 and T2 are not identical, wherein FOR and FOR1 are not identical and wherein REV and REV1 are not identical;
b) Transferring said sample into a vial V1;
c) Providing a plasmid comprising a control sequence 1 (S1) and a control sequence 3 (S3), wherein S1 is flanked by a sequence hybridizing to FOR and a sequence hybridizing to REV, wherein S3 is flanked by a sequence hybridizing to FOR1 and a sequence hybridizing to REV1, and wherein T1, T2, S1 and S3 are not identical;
d) Transferring said plasmid of step c) into a vial V2;
e) Providing a plasmid comprising a control sequence 2 (S2), wherein S2 is flanked by a sequence hybridizing to FOR and a sequence hybridizing to REV, wherein T, S1 and S2 are not identical;
f) Transferring said plasmid step e) into said vials;
g) Extracting nucleic acids in said vials;
h) Conducting PCR reactions in said vials using the primers FOR, REV, FOR1 and REV1;
i) Sequencing the nucleic acids amplified in said vials;
wherein the presence of T1 and T2 in vial V1 indicates the presence of said nucleic acid comprising T1 and T2 in said sample if the sequencing in vial V2 resulted in the presence of S1, S2 and S3, and if the sequencing in vial V1 resulted in the presence of T1, T2 and S2.

The setup described above may thus be used to characterize a specific nucleic acid by detecting the presence of at least two target sequences in said nucleic acid. This setup may particularly be used to increase the specificity of the method.

Also disclosed is plasmid comprising a control sequence (S), wherein S is flanked by a sequence hybridizing to a forward primer (FOR) and a sequence hybridizing to a reverse primer (REV), wherein said sequences hybridizing to FOR and REV are sequences derived from a nucleic acid comprising said sequence hybridizing to FOR, followed by a target sequence (T), followed by said sequence hybridizing to REV, and wherein S and T are not identical.

In a preferred embodiment, the hybridization of FOR and REV to said plasmid during an amplification reaction results in the amplification of S.

Said nucleic acid comprising said sequence hybridizing to FOR, followed by T, followed by said sequence hybridizing to REV may be a nucleic acid from a microorganism.

Preferably, said microorganism is selected from the group consisting of bacteria, archaea, protozoa, fungi and viruses. In a particularly preferred embodiment, said microorganism is a bacterium selected from the group consisting of Group A Streptococcus, *Mycobacterium tuberculosis* Complex members including *Mycobacterium tuberculosis, Mycobacterium africanum, Mycobacterium bovis, Mycobacterium canetti* and *Mycobacterium microti, Salmonella enterica* spp., *Clostridium difficile,* Vancomycin-resistant enterococcus and Methicillin-resistant *Staphylococcus aureus.* In another particularly preferred embodiment, said microorganism is a virus selected from the group consisting of adenovirus, avian influenza A (H7N9) virus, Middle East Respiratory Syndrome Coronavirus, norovirus, BK virus, cytomegalovirus, Epstein-Barr virus, herpex simplex virus 1, herpex simplex virus 2, Varicella-Zoster virus, enterovirus, human immunodeficiency virus (HIV), in particular HIV-1, hepatitis B virus (HBV), hepatitis C virus (HCV), Dengue virus and Chikungunya virus. In the most preferred embodiment, said microorganism is a virus selected from the group consisting of HIV (in particular HIV-1), HBV and HCV.

Said nucleic acid comprising T may be an oncogene, preferably a human oncogene. Preferably, said human oncogene is selected from the group consisting of BCR-ABL major, BCR-ABL minor, BCR-AML1 ETO, PML-RARA, BRAF V600 mutants, KRAS mutants and NRAS mutants.

In said plasmid, S has no homology to T and is selected such that the sequence is amplified using the reagents and conditions for amplification of T (this applies e.g. to the G/C-content of S). S may e.g. be derived from the genome of a plant virus, in particular of the tobacco mosaic virus (TMV). However, S may also be an artificial sequence, which is not naturally occurring.

T and S can have a length of less than or equal to about 2000 bases. Particularly preferred is a length of less than or equal to about 1000 bases, preferably of about 600 bases or about 400 bases.

Said plasmid may be a prokaryotic or eukaryotic plasmid. It can be particularly preferred to use a prokaryotic plasmid, in particular a commonly used *E. coli* plasmid.

Such a plasmid may be used in step c) of the method according to the first aspect of the invention and serves as positive and negative control.

Such a plasmid may be used in step e) of the method according to the first aspect of the invention and serves as extraction control.

Also disclosed is the use of a plasmid in a sequencing assay designed to detect the presence of T in a sample in a sample reaction (SR), wherein said plasmid is used in a control reaction (CR) and wherein CR and SR are separate reactions.

Further disclosed is the use of a plasmid in a sequencing assay designed to detect the presence of T in a sample in a sample reaction (SR), wherein said plasmid is added to said SR prior to the extraction of nucleic acids therefrom.

Therefore, a plasmid is also disclosed comprising a control sequence 1 (S1), wherein S1 is flanked by a sequence hybridizing to a forward primer (FOR) and a sequence hybridizing to a reverse primer (REV), a control sequence 2 (S2), wherein S2 is flanked by sequence hybridizing to a forward primer (FOR1) and a sequence hybridizing to a reverse primer (REV1), wherein said sequences hybridizing to FOR, FOR1, REV and REV1 are sequences derived from a nucleic acid comprising said sequence hybridizing to FOR, followed by a target sequence 1 (T1), followed by said sequence hybridizing to REV, and said sequence hybridizing to FOR1, followed by a target sequence 2 (T2), followed by said sequence hybridizing to REV1, and wherein S1, S2, T1 and T2 are not identical. Said plasmid preferably serves as both, positive and negative control.

In a further aspect, the present invention relates to a kit for the detection of a nucleic acid comprising a target sequence (T) in a sample, wherein said kit comprises
(a) primers FOR and REV, wherein FOR and REV hybridize to sequences flanking T;
(b) a plasmid comprising a sequence hybridizing to FOR, followed by a control sequence 1 (S1), followed by a sequence hybridizing to REV;
(c) a plasmid comprising a sequence hybridizing to FOR, followed by a control sequence 2 (S2), followed by a sequence hybridizing to REV;
wherein T, S1 and S2 are not identical.

In an embodiment thereof, the present invention relates to a kit for the detection of a nucleic acid comprising target sequences 1 and 2 (T1 and T2) in a sample, wherein said kit comprises
(a) primers FOR and REV, wherein FOR and REV hybridize to sequences flanking T1;
(b) primers FOR1 and REV1, wherein FOR1 and REV1 hybridize to sequences flanking T2;
(c) a plasmid comprising a sequence hybridizing to FOR, followed by a control sequence 1 (S1), followed by a sequence hybridizing to REV, and a sequence hybridizing to FOR1, followed by a control sequence 3 (S3), followed by a sequence hybridizing to REV1;
(d) a plasmid comprising a sequence hybridizing to FOR, followed by a control sequence 2 (S2), followed by a sequence hybridizing to REV;
wherein T1, T2, S1, S2 and S3 are not identical, wherein FOR and FOR1 are not identical and wherein REV and REV1 are not identical.

The present invention also discloses the following kit: a kit for the detection of a nucleic acid comprising a target sequence (T) in a sample, wherein said kit comprises
(a) primers FOR and REV, wherein FOR and REV hybridize to sequences flanking T;
(b) a plasmid comprising a sequence hybridizing to FOR, followed by a control sequence 1 (S1), followed by a sequence hybridizing to REV;
wherein T and S1 are not identical.

In an embodiment thereof, the present invention relates to a kit for the detection of a nucleic acid comprising target sequences 1 and 2 (T1 and T2) in a sample, wherein said kit comprises
(a) primers FOR and REV, wherein FOR and REV hybridize to sequences flanking T1;
(b) primers FOR1 and REV1, wherein FOR1 and REV1 hybridize to sequences flanking T2;
(c) a plasmid comprising a sequence hybridizing to FOR, followed by a control sequence 1 (S1), followed by a sequence hybridizing to REV, and a sequence hybridizing to FOR1, followed by a control sequence 2 (S2), followed by a sequence hybridizing to REV1;
wherein T1, T2, S1 and S2 are not identical, wherein FOR and FOR1 are not identical and wherein REV and REV1 are not identical.

In a preferred embodiment, the kits as mentioned above comprise instructions for use.

Finally, the present invention relates to the use of the kits according to the third aspect of the present invention in a method for the detection of a nucleic acid comprising a target sequence (T) or a nucleic acid comprising target sequences 1 and 2 (T1 and T2), respectively, in a sample.

### DESCRIPTION OF THE FIGURES

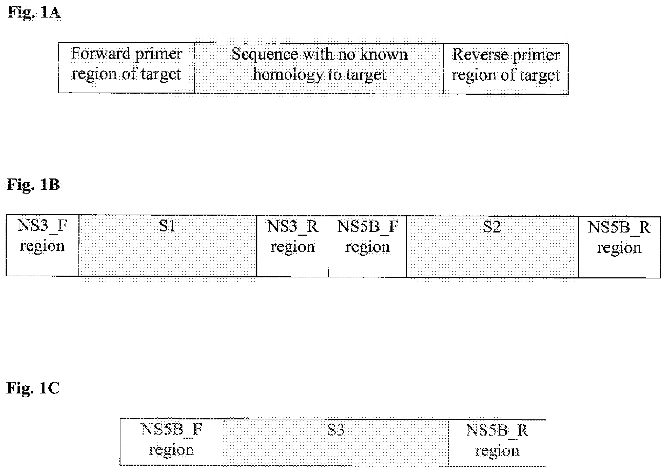

Figure 1A shows a schematic view of the sequences comprised in a plasmid according to the present invention in the direction 5' to 3': a sequence hybridizing to a forward primer, followed by a control sequence, followed by a region hybridizing to a reverse primer.
Figure 1B shows a schematic view of two control sequences comprised in a plasmid used as positive and negative control according to the present invention, again in the direction 5' to 3'. The specific example relates to two genes comprised in the HCV nucleic acid, namely the NS3 and NS5B regions: a sequence hybridizing to an NS3-forward primer (used for the amplification of NS3 in a sample), a control sequence S1, followed by a sequence hybridizing to an NS3-reverse primer (used for the amplification of NS3 in a sample); followed by a sequence hybridizing to an NS5B-forward primer (used for the amplification of NS5B in a sample), a control sequence S2, followed by a sequence hybridizing to an NS5B-reverse primer (used for the amplification of NS5B in a sample).
Figure 1C shows a schematic view of the sequences comprised in a plasmid used as extraction control according to the present invention, again in the direction 5' to 3'. The specific example relates to a gene comprised in the HCV nucleic acid, namely the NS5B regions: a sequence hybridizing to an NS5B-forward primer (used for the amplification of NS5B in a sample), a control sequence S3, followed by a sequence hybridizing to an NS5B-reverse primer (used for the amplification of NS5B in a sample).

### DETAILED DESCRIPTION OF THE INVENTION

The inventors of the present invention *inter alia* succeeded in providing universal sequencing controls, which may be used as positive and negative as well as extraction control in a sequencing assay.

Before some of the embodiments of the present invention are described in more detail, the following definitions are introduced.

### Definitions

As used in the specification and the claims, the singular forms of "a" and "an" also include the corresponding plurals unless the context clearly dictates otherwise.

The term "about" in the context of the present invention denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present invention, the term "consisting of' is considered to be a preferred embodiment of the term "comprising". If hereinafter a group is defined to comprise at least a certain number of embodiments, this is also meant to encompass a group which preferably consists of these embodiments only.

The term "detecting the presence" as used herein is to be understood in the meaning of "detecting the presence or absence". As mentioned in the method as claimed in the present application, the sample to be analyzed *potentially* comprises a nucleic acid comprising a target sequence. Thus, there may e.g. be indications that a patient is infected with a hepatitis C virus and a corresponding blood sample potentially comprising an HCV nucleic acid is analyzed by a method according to the present invention. Assuming that all controls indicate that the assay has been carried out properly, the result is that the target sequence(s) (and thus the virus) is(are) either present or absent - accordingly, the presence or absence of the target sequence in said sample is detected.

In the context of the present invention the term "nucleic acid" refers to a naturally occurring deoxyribonucleotide or ribonucleotide polymer in either single-or double-stranded form. The nucleic acid may particularly be double-stranded DNA and single-stranded RNA.

The term "sequence" as used herein refers to the sequential occurrence of the bases in a deoxyribonucleotide or ribonucleotide polymer, wherein a base found in a deoxyribonucleotide polymer is selected from the group consisting of A, T, G and C and a base found in a ribonucleotide polymer is selected from the group consisting of A, U, G and C. A sequence of bases in a deoxyribonucleotide polymer may thus e.g. be GGAAGCAAGCCT (SEQ ID No.:14), whereas a sequence of bases in a ribonucleotide polymer may e.g. be GGAAUCGAUAU (SEQ ID No:15).

A "target sequence" as referred to herein is a sequence in the nucleic acid, the presence of which is detected in the method according to the present invention; a "target sequence" is characteristic for the specific nucleic acid, the presence of which is detected. If e.g. an HCV nucleic acid is detected, the target sequence may e.g. comprise the NS3 and/or the NS5A and/or the NS5B genes of HCV (see also examples 1 and 2).

As used herein, the term "sample" refers to any biological sample from any human or veterinary subject that may be tested for the presence of a nucleic acid comprising a target sequence. The samples may include tissues obtained from any organ, such as for example, lung tissue; and fluids obtained from any organ such as for example, blood, plasma, serum, lymphatic fluid, synovial fluid, cerebrospinal fluid, amniotic fluid, amniotic cord blood, tears, saliva, and nasopharyngeal washes. As listed above, samples may also be derived from a specific region in the body, e.g. the respiratory tract; samples from the respiratory tract include throat swabs, throat washings, nasal swabs, and specimens from the lower respiratory tract.

The sample may in particular be derived from a human or a veterinary subject. Accordingly, a "patient" may be a human or veterinary subject. If reference is made to a "clinical sample", this indicates that the sample is from a patient suspicious of carrying a nucleic acid comprising a target sequence.

The term "flanked" as used herein in connection with sequences means that the two sequences described as flanking a specific sequence (e.g. a sequence hybridizing to a forward primer and a sequence hybridizing to a reverse primer) are comprised upstream and downstream of said specific sequence. If reference is made in this context to a sequence hybridizing to a forward primer, this region lies upstream, i.e. at the 5'-end of said sequence. Following the target sequence, a sequence hybridizing to a reverse primer is then present, i.e. downstream of said sequence or at the 3'-end. This setup can also be derived from Figure 1A.

The term "primer" refers to an oligonucleotide that is capable of acting as a point of initiation for the 5' to 3' synthesis of a primer extension product that is complementary to a nucleic acid strand. The primer extension product is synthesized in the presence of appropriate nucleotides and an agent for polymerization such as a DNA polymerase in an appropriate buffer and at a suitable temperature. Primers can be designed using, for example, a computer program such as OLIGO (Molecular Biology Insights, Inc., Cascade, Colorado). Important features when designing primers include an appropriate size of the amplification product, preferably in the ranges set out above, to facilitate detection, similar melting temperatures for the members of a pair of primers, and the length of each primer (i.e., the primers need to be long enough to anneal with sequence-specificity and to initiate synthesis but not so long that fidelity is reduced during oligonucleotide synthesis). Typically, primers are 15 to 30 nucleotides in length.

A "forward primer" hybridizing to a region upstream of a specific sequence and a "reverse primer" hybridizing to a region downstream of a specific sequence will hybridize such that said specific sequence will be amplified during a PCR amplification reaction. If double stranded DNA or cDNA is present in the sample, the forward primer will hybridize to the upstream region such that its 3'-end points towards the sequence to be amplified; the 3'-end of the reverse primer also points to the sequence to be amplified. As in every PCR setup, the primers will thus hybridize to different strands: the forward primer hybridizes to the noncoding strand, whereas the reverse primer hybridizes to the coding strand.

As used herein, the term "amplification" refers to enzyme-mediated procedures that are capable of producing billions of copies of nucleic acid target. Examples of enzyme-mediated target amplification procedures known in the art include PCR.

As used herein the term "hybridizing" refers to the process of establishing a noncovalent, sequence-specific interaction between two or more complementary strands of single-stranded nucleic acids into a complex, preferably a duplex in the present invention.

The term "vial" refers to a reaction vial as typically used in diagnostic assays. The vial may be comprised on a multiplate, e.g. a 96-well plate and may thus also be referred to as "well", or it may be comprised on a sample ring, e.g. a 36-vial sample ring. If an object is "transferred" into a vial, as sterile conditions as possible are preferably used in this step; in some setups, the transfer step may be carried out by pipetting the object into a vial; this may be done in an automated manner. A typical "object" in the present context is a clinical sample, a plasmid comprised in buffer, or cells comprising a plasmid. If cells comprising a plasmid are transferred into a vial, this may also be done by adding said cells to a specific buffer used during an extraction process, e.g. a lysis buffer.

The term "plasmid" is used as herein according to its standard meaning in molecular biology. Any type of prokaryotic or eukaryotic vector may be used for the purposes of the present invention, i.e. for the plasmid used as positive and negative control as well as the extraction control. Examples of such plasmids are TMV plasmids.

The term "identical" as used herein in connection with "not identical sequences" means that the sequences differ in at least one base from each other. As noted above, it is, however, preferred that the sequences show no homology at all, at least as regards the target sequence(s) and the control sequence(s). Different control sequences may show some degree of homology but clearly must be distinguishable from each other. This is achieved in that the control sequences also differ from each other by at least one base. In a preferred embodiment, T thus shows no homology to S1 and S2, wherein S1 and S2 may share some homology but differ in at least one base, preferably more than one base from each other.

"Extracting nucleic acids" means that any nucleic acids present in a vial are substantially isolated from any cellular background, particularly isolated from intact cells. Preferably, the nucleic acids are also washed during the process and optionally concentrated. Following an extraction, substantially all intact cells present in a vial have been lysed and substantially all cellular debris not related to nucleic acids has been removed. Typical extraction methods may include the use of hypotonic lysis buffer, heat and/or detergents, and are known to the skilled person. A particularly preferred extraction process according to the present invention comprises the following steps:
- proteinase K is added to a commonly used lysis buffer; if the subsequent steps comprise the step of carrying out a reverse transcription (via RT-PCR), carrier RNA may also be added to the lysis buffer; further, as outlined above, the extraction control plasmid may also be added to the lysis buffer ("spiked into the lysis buffer");
- the lysis buffer described above is added to the samples and the samples are incubated for 10 minutes at 60°C, preferably while the samples are mixed;
- magnetic beads commonly used for capturing nucleic acids are preferably used in order to isolate nucleic acids from the samples (according to standard protocols); the next steps may thus comprise:
   ∘ binding buffer is added to the samples after lysis of the cells;
   ∘ the magnetic beads for capturing nucleic acids are added;
   ∘ the magnetic beads are collected via a magnet and the supernatant is removed (preferably after a short incubation);
   ∘ optionally several different (preferably two) wash buffers are added to the beads and several (preferably two) wash steps are carried out sequentially by washing the beads (mixing, collecting the beads via a magnet and removing the supernatant; adding the next wash buffer);
   ∘ the beads are then typically dried;
   ∘ elution buffer is added to the beads and the samples are typically mixed, preferably after a short incubation;
   ∘ the magnetic beads are collected via a magnet and the supernatant comprising the eluted nucleic acids is collected;
- the supernatant comprising the eluted nucleic acids (corresponding to the "extracted nucleic acids") is used in the subsequent steps.

A "PCR reaction" has first been described for the amplification of DNA by Mullis et al. in U.S. Patent No. 4,683,195 and Mullis in U.S. Patent No. 4,683,202 and is well known to those of ordinary skill in the art. In the PCR technique, a sample of DNA is mixed in a solution with a molar excess of at least two oligonucleotide primers of that are prepared to be complementary to the 3' end of each strand of the DNA duplex (see above, a forward and a reverse primer); a molar excess of nucleotide bases (i.e., dNTPs); and a heat stable DNA polymerase, (preferably Taq polymerase), which catalyzes the formation of DNA from the oligonucleotide primers and dNTPs. Of the primers, at least one is a forward primer that will bind in the 5' to 3' direction to the 3' end of one strand (in the above definition the non-sense strand) of the denatured DNA analyte and another is a reverse primer that will bind in the 3' to 5' direction to the 5' end of the other strand (in the above definition the sense strand) of the denatured DNA analyte. The solution is heated to about 94-96°C to denature the double- stranded DNA to single-stranded DNA. When the solution cools down and reaches the so-called annealing temperature, the primers bind to separated strands and the DNA polymerase catalyzes a new strand of analyte by joining the dNTPs to the primers. When the process is repeated and the extension products synthesized from the primers are separated from their complements, each extension product serves as a template for a complementary extension product synthesized from the other primer. As the sequence being amplified doubles after each cycle, a theoretical amplification of a huge number of copies may be attained after repeating the process for a few hours; accordingly, extremely small quantities of DNA may be amplified using PCR in a relatively short period of time.

Where the starting material for the PCR reaction is RNA, complementary DNA ("cDNA") is synthesized from RNA via reverse transcription. The resultant cDNA is then amplified using the PCR protocol described above. Reverse transcriptases are known to those of ordinary skill in the art as enzymes found in retroviruses that can synthesize complementary single strands of DNA from an mRNA sequence as a template. A PCR used to amplify RNA products is referred to as reverse transcriptase PCR or "RT-PCR".

The terms "complementary" and "substantially complementary" as used in the above definitions refer to base pairing between nucleotides or nucleic acids, such as, for instance, between the two strands of a double-stranded DNA molecule or between an oligonucleotide primer and a primer binding site on a single- stranded nucleic acid to be sequenced or amplified. Complementary nucleotides are, generally, A and T (or A and U), and G and C.

The term "sequencing" is used herein in its common meaning in molecular biology. Thus, the exact sequential occurrence of bases in a nucleic acid sequence is determined.

The term "microorganism" as used herein is used in its broadest meaning. Thus, a microorganism may be any type of bacteria, archaeum, protozoum, fungus and virus. It is explicitly mentioned that viruses fall under the definition of a "microorganism" as used herein.

The term "oncogene" is used herein in its common meaning in molecular biology and oncology, respectively. Thus, there are e.g. mutations known in genes, which render a "normal or wild-type" gene oncogenic, i.e. cancer-inducing; examples in this respect are mutations rendering kinases constitutionally active such that specific signals (e.g. growth inducing signals) are constantly signaled and corresponding processes initiated. "Oncogenes" as used herein may also relate to intra- or inter-chromosomal translocations resulting also in cancer-inducing situations.

The term "multiplex" refers to the detection of the presence of a specific nucleic acid in several samples, wherein the corresponding assays are carried out simultaneously, i.e. the steps of the present method are generally performed in parallel.

The following examples are put forth so as to provide those of ordinary skill in the art with a complete disclosure and description of how to make and use the compositions of the invention. The examples are intended as non-limiting examples of the invention. While efforts have been made to ensure accuracy with respect to variables such as amounts, temperature, etc., experimental error and deviations should be taken into account. Unless indicated otherwise, parts are parts by weight, temperature is degrees centigrade, and pressure is at or near atmospheric. All components were obtained commercially unless otherwise indicated.

### Examples

### Example 1: A plasmid to be used as positive and negative control

The goal of the present assay (as described in Example 3 in more detail) is the detection of the presence or absence of a nucleic acid of the hepatitis C virus (HCV) in a clinical sample. There are two target sequences to be detected in the HCV nucleic acid; these sequences are comprised in the genes NS3 and NS5B of HCV.

A plasmid to be used as positive and negative control comprises the following sequences (in 5' to 3' direction, see also Figure 1B):
- A primer region derived from the NS3 HCV gene (highlighted in light grey), wherein the sequence hybridizing with the NS3-forward primer is underlined (note: the forward primer hydridizes to the complementary strand of the strand shown below during an amplification reaction); SEQ ID No.:1
- A sequence derived from the tobacco mosaic virus (TMV), which shows no homology to the HCV NS3 gene (referred to as S1 in the following); SEQ ID No.:2
- A primer region derived from the NS3 HCV gene (highlighted in light grey), wherein the sequence hybridizing with the NS3-reverse primer is underlined (note: the reverse primer hydridizes to the strand shown below during an amplification reaction); SEQ ID No.:3
- A primer region derived from the NS5B HCV gene (highlighted in dark grey), wherein the sequence hybridizing with the NS5B-forward primer is underlined (note: the forward primer hydridizes to the complementary strand of the strand shown below during an amplification reaction); SEQ ID No.:4
- A second sequence derived from TMV (S2), which shows no homology to the HCV NS5B gene and which differs from S1; SEQ ID No.:5
- A primer region derived from the NS5B HCV gene (highlighted in dark grey), wherein the sequence hybridizing with the NS5B-reverse primer is underlined (note: the reverse primer hydridizes to the strand shown below during an amplification reaction); SEQ ID No.:6

An exemplary sequence comprising the above elements is the following (SEQ ID No.:7):

GCACAACGGCCTGCGAGATCTGGCCGTGGCTGTGGAACCAGTCGTCTTCT
CCCGAATGGAGACCAAGCTCATCACGTGGGGGCAGATACCGCCGCGTG
CGGTGACATCATCAACGGCTTGCCCGTCTCTGCCCGTA
ggaagcaagccgcggaaatgatcagaagacgtgcgaattcctcagggattattgtggcca
cgaaggataacgttaaaaccgttgattctttcatgatgaattttgggaaaagcacacgct
gtcagttcaagaggttattcattgatgaagggttgatgttgcatactggttgtgttaatt
ttcttgtggcgatgtcattgtgcgaaattgcatatgtttacggagacacacagcagattc
catacatcaatagagtttcaggattcccgtaccccgcccattttgccaaattggaggttg
acgaggtggagacacgcagaactactctccgttgtccagccgatgtcacacattatctga
acaggagatatgagggctttgtcatgagcacttcttcggttaaaaagtctgtttcgcagg
agatggtcggcggagccgccgtgatcaatccgatctcaaaacccttgcatggcaagatcc
tgacttttacccaatcggataaagaagctctgctttcaagagggtattcagatgttcaca
ctgtgcatgaagtgcaaggcgagacatactctgatgtttcactagttaggttaacccccta
CAGGACCGGGGTGAGAACAATTACCACTGGCAGCCCCATCACGTACTCC
ACCTACGGCAAGTTCCTTGCCGACGGCGGGTGCTCAGGAGGTGCTTATGA
CATAATAATTTGTGACGAGTGCCACTCCACGGATGCCACAT
TGCACCATGCTCGTGTGTGGCGACGACTTAGTCGTTATCTGTGAAAGTGC
GGGGGTCCAGGAGGACGCGGCGAGCCTGAGAGCCTTCACGGAGGCTATG
ACCAGGTACTCCGCCCCCCCGGGGACCCCCCACAACC
gcatattggatatgtctaagtctgttgctgcgcctaaggatcaaatcaaaccactaatac
ctatggtacgaacggcggcagaaatgccacgccagactggactattggaaaatttagtgg
cgatgattaaaaggaactttaacgcacccgagttgtctggcatcattgatattgaaaata
ctgcatctttggttgtagataagttttttgatagttatttgcttaaagaaaaaagaaaac
caaataaaaatgtttctttgttcagtagagagtctctcaatagatggttagaaaagcagg
aacaggtaacaattggccagctcgcagattttgattttgtggatttgccagcagttgatc
agtacagacacatgattaaagcacaacccaagcaaaagttggacacttcaatccaaacgg
agtacccggctttgcagacgattgtgtaccattcaaaaaagatcaatgcaatattcggcc
cgttgtttagcgagcttactaggcaattactggacagtgttgattcgagcagattttgt

ttttcacaagaaagacaccagcgcagattgaggatttcttcggagatctcgacagtcatg
GGCTGGACTTGTCCGGTTGGTTCACGGCTGGCTACAGCGGGGAGACATT
TATCACAGCGTGTCTCATGCCCGGCCCCGCTGGTTCTGGTTTTGCCTACTC
CTGCTCGCTGCAGGGGTAGGCATCTACCTCCTCCCCAACCGATGA

The assay is carried out as described in example 3. Successful detection of the sequences S1 and S2 in the control vial (comprising the plasmid comprising the sequences shown above) will indicate that the PCR reactions using primer pairs NS3-for and NS3-rev and NS5B-for and NS5B-rev, respectively, worked properly (positive control). If S1 and S2 were detected, it can be excluded that the samples were contaminated (e.g. with a sample comprising the virus or the virus itself) since otherwise the sequences of the HCV genes NS3 and NS5B would also have been detected in this control (negative control). No additional negative control is required. As discussed in example 2, an extraction control plasmid is usually also added to the vial.

### Example 2: A plasmid to be used as extraction control

The general setup is identical to the setup outlined above. Thus, the goal of the assay is the detection of the presence or absence of a nucleic acid from the hepatitis C virus (HCV) in a clinical sample, wherein HCV-sequences comprised in the genes NS3 and NS5B of HCV are detected.

A plasmid to be used as extraction control comprises the following sequences (in 5' to 3'-direction, see also Figure 1C):
- A primer region derived from the NS5B HCV gene (highlighted in light grey), wherein the sequence hybridizing with the NS5B-forward primer is underlined (note: the forward primer hydridizes to the complementary strand of the strand shown below during an amplification reaction); SEQ ID No.:1
- A second sequence derived from TMV (S3), which shows no homology to the HCV NS5B gene and which differs from S1 and S2; SEQ ID No.:8
- A primer region derived from the NS5B HCV gene (highlighted in light grey), wherein the sequence hybridizing with the NS5B-reverse primer is underlined (note: the reverse primer hydridizes to the strand shown below during an amplification reaction); SEQ ID No.:3.

An exemplary sequence is the following (SEQ ID No.:9):

GCACAACGGCCTGCGAGATCTGGCCGTGGCTGTGGAACCAGTCGTCTTCT
CCCGAATGGAGACCAAGCTCATCACGTGGGGGCAGATACCGCCGCGTG
CGGTGACATCATCAACGGCTTGCCCGTCTCTGCCCGTA
gcatctggtatcaaagaaagagcgggggacgtcacgacgttcattggaaacactgtgatca
ttgctgcatgtttggcctcgatgcttccgatggagaaaataatcaaaggagccttttgtg
gtgacgatagtctgctgtactttccaaagggttgtgagtttccggatgtgcaacactccg
cgaatcttatgtggaattttgaagcaaaactgtttaaaaaacagtatggatacttttgcg
gaagatatgtaatacatcacgacagaggatgcattgtgtattacgatcccctaaagttga
tctcgaaacttggtgctaaacacatcaaggattgggaacacttggaggagttcagaaggt
ctctttgtgatgttgctgtttcgttgaacaattgtgcgtattacacacagttggacgacg
ctgtatgggaggttcataaaaccgcccctccaggttcgtttgtttataaaagtctggtga
agtatttgtctgataaagtt
CAGGACCGGGGTGAGAACAATTACCACTGGCAGCCCCATCACGTACTCC
ACCTACGGCAAGTTCCTTGCCGACGGCGGGTGCTCAGGAGGTGCTTATGA
CATAATAATTTGTGACGAGTGCCACTCCACGGATGCCACAT

The assay is carried out as described in Example 3, wherein a standardized amount of the extraction control plasmid is spiked into the lysis buffer used in the extraction step of all samples including the control of example 2.

Successful detection of the HCV target sequences and the sequence S3 in a vial comprising a sample (and the above plasmid) will at least indicate that the extraction step worked properly (extraction control). If only the HCV target sequences are detected, the extraction step was not carried out properly - to the contrary, such a result is indicative of a contamination of the samples with viral DNA. No additional primer pair is required for the amplification reaction of the sequence comprised in the cells of the extraction control.

### Example 3: Assay using the plasmids of example 1 and 2

The assay described in the following is carried out in order to determine the presence or absence of a nucleic acid of the hepatitis C virus (HCV) in a clinical sample such as blood from a human subject.

The assay is carried out using the Sentosa SX101 device by Vela Diagnostics, the Rotor-Gene Q device by Qiagen and an Ion Torrent Semiconductor Sequencing device by life technologies.

The clinical sample to be analyzed is provided in a suitable vial or well; further samples may be provided as well (of course in different vials), wherein all samples can be analyzed in parallel. The plasmid of Example 1 is placed into another vial in an appropriate concentration and volume. The samples in all vials / wells (including the vial comprising the plasmid of Example 1) are in a first step subject to the automated extraction procedure of the Sentosa SX101 device. This procedure uses the plasmid of Example 2 in the lysis buffer; thus, the nucleic acids in all vials are then extracted.

In the next step, the samples are automatically transferred to a sample ring. Such a sample ring may comprise up to 72 vials, wherein the positive control (i.e. the plasmid of Example 1) may be transferred into vial 1 and the samples may be transferred into vials 2 to 72.

The Sentosa SX101 device is also capable of automatically loading the samples with the components used in the next step - in the present example, the next step is an RT-PCR since HCV is an RNA virus. Therefore, an initial RT-PCR needs to be carried out in the regions of the NS3- and NS5B-genes to be detected. The corresponding components are added to the extracted nucleic acids in all vials, i.e. vials 1 to 72. The components comprise the enzymes reverse transcriptase and Taq polymerase as well as the following primers:

| | |
|---|---|
| NS3_fw: | TGGGGGGCAGATACCGC (SEQ ID No.:10) |
| NS3_rev: | AGGAACTTGCCGTAGGTGGAGTA (SEQ ID No.:11) |
| NS5B_fw: | CCTTCACGGAGGCTATGACCAGGTA (SEQ ID No.:12) |
| NS5B_rev: | TGAGACACGCTGTGATAAATGTC (SEQ ID No.:13) |

The sample ring comprising the extracted nucleic acids together with all components required for an RT-PCR is then transferred to a Rotor-Gene Q device, where the RT-PCR reactions are carried out according to a standard protocol.

The samples are then transferred from the individual vials to a microwell used in an Ion Torrent Semiconductor Sequencing device. The sequencing (including an optionally necessary step of fragmenting the amplified nucleic acids) is carried out according to a standard protocol.

The plasmid according to example 1 was comprised in vial 1; as noted above, an extraction step was also carried out for this vial - thus, lysis buffer comprising the plasmid of example 2 was added to this vial. A successful detection of sequences S1 and S2 indicates that the PCR reactions were carried out properly. The plasmid according to Example 1 thus serves as positive control for the PCR-reaction. Since the plasmid of example 2 was also added, sequence S2 should also be detected - if no further sequences apart from S1, S2 and S3 are detected in vial 1, a contamination of the samples can be excluded. The reaction thus also serves as negative control. As noted above, the plasmid according to example 2 was added to the lysis buffer used for the extraction of the nucleic acids in all samples. Successful extraction of the nucleic acids is indicated by the presence of sequence S3 in all vials.
If the sequences to be detected in HCV, i.e. the regions in genes NS3 and NS5B, are also (in addition to sequence S3) present in vial 2 (and, if applicable, in all further sample vials), nucleic acids derived from HCV were indeed present. This is indicative of the presence of HCV in the clinical sample(s).

If only sequence S3 was detected in vial 2, no nucleic acids from HCV were present in the sample; accordingly, an HCV infection can be excluded.

## Claims

1. An *in vitro* method of detecting the presence of a nucleic acid comprising a target sequence (T) in a sample comprising the following steps:
a) Using a sample potentially comprising a nucleic acid comprising T, wherein T is flanked by a sequence hybridizing to a forward primer (FOR) and a sequence hybridizing to a reverse primer (REV);
b) Transferring said sample into a vial V1;
c) Providing a plasmid comprising a control sequence 1 (S1), wherein S1 is flanked by a sequence hybridizing to FOR and a sequence hybridizing to REV, wherein T and S1 are not identical;
d) Transferring said plasmid of step c) into a vial V2;
e) Providing a plasmid comprising a control sequence 2 (S2), wherein S2 is flanked by a sequence hybridizing to FOR and a sequence hybridizing to REV, wherein T, S1 and S2 are not identical;
f) Transferring said plasmid of step e) into said vials;
g) Extracting nucleic acids in said vials;
h) Conducting PCR reactions in said vials using the primers FOR and REV;
i) Sequencing the nucleic acids amplified in said vials;
wherein the presence of T in vial V1 indicates the presence of said nucleic acid comprising T in said sample if the sequencing in vial V2 resulted in the presence of S1 and S2, and if the sequencing in vial V1 resulted in the presence of T and S2.

2. Method according to claim 1, wherein said sample is a clinical sample.

3. Method according to claim 2, wherein said clinical sample is a tissue sample or a body fluid sample, preferably from a human subject.

4. Method according to claim 1, wherein said nucleic acid comprising T is a nucleic acid from a microorganism.

5. Method according to claim 3, wherein said microorganism is selected from the group consisting of bacteria, archaea, protozoa, fungi and viruses.

6. Method according to claim 1, wherein said nucleic acid comprising T is an oncogene, preferably a human oncogene.

7. A kit for the detection of a nucleic acid comprising a target sequence (T) in a sample, wherein said kit comprises
(a) primers FOR and REV, wherein FOR and REV hybridize to sequences flanking T;
(b) a plasmid comprising a sequence hybridizing to FOR, followed by a control sequence 1 (S1), followed by a sequence hybridizing to REV;
(c) a plasmid comprising a sequence hybridizing to FOR, followed by a control sequence 2 (S2), followed by a sequence hybridizing to REV;
wherein T, S1 and S2 are not identical.

## Patentansprüche

1. In vitro-Verfahren zum Nachweisen des Vorhandenseins einer eine Zielsequenz (T) umfassenden Nukleinsäure in einer Probe das die folgenden Schritte umfasst:
a) Verwenden einer Probe, die potenziell eine Nukleinsäure umfasst, welche T umfasst, wobei T flankiert wird von einer Sequenz, die an einen Vorwärtsprimer (FOR) hybridisiert, und von einer Sequenz, die an einen Umkehrprimer (REV) hybridisiert;
b) Überführen der Probe in ein Gefäß V1;
c) Bereitstellen eines Plasmids, das eine Kontrollsequenz 1 (S1) umfasst, wobei S1 von einer Sequenz flankiert wird, die an FOR hybridisiert und von einer Sequenz, die an REV hybridisiert, wobei T und S1 nicht identisch sind;
d) Überführen des Plasmids aus Schritt c) in ein Gefäß V2;
e) Bereitstellen eines Plasmids, das eine Kontrollsequenz 2 (S2) umfasst, wobei S2 von einer Sequenz flankiert ist, die an FOR hybridisiert und von einer Sequenz, die an REV hybridisiert, wobei T, S1 und S2 nicht identisch sind;
f) Überführen dieses Plasmids aus Schritt e) in die Gefäße;
g) Extrahieren von Nukleinsäuren in diesen Gefäßen;
h) Durchführen von PCR Reaktionen in diesen Gefäßen unter Verwendung der Primer FOR und REV;
i) Sequenzieren der in diesen Gefäßen amplifizierten Nukleinsäuren;
wobei das Vorhandensein von T in Gefäß V1 das Vorliegen der Nukleinsäure, die T umfasst, in der Probe anzeigt, sofern die Sequenzierung in Gefäß V2 zum Vorliegen von S1 und S2 führt, und sofern die Sequenzierung in Gefäß V1 zum Vorliegen von T und S2 führt.

2. Verfahren gemäß Anspruch 1, wobei die Probe eine klinische Probe ist.

3. Verfahren gemäß Anspruch 2, wobei die klinische Probe eine Gewebeprobe ist oder eine Probe einer Körperflüssigkeit, vorzugsweise eines menschlichen Individuums.

4. Verfahren gemäß Anspruch 1, wobei die Nukleinsäure, die T umfasst, eine Nukleinsäure eines Mikroorganismus ist.

5. Verfahren gemäß Anspruch 3, wobei der Mikroorganismus aus der Gruppe ausgewählt ist, die aus Bakterien, Archaeen, Protozoen, Pilzen und Viren besteht.

6. Verfahren gemäß Anspruch 1, wobei die Nukleinsäure, die T umfasst, ein Oncogen ist, vorzugsweise ein menschliches Oncogen.

7. Kit zum Nachweis einer Nukleinsäure, die eine Zielsequenz (T) in einer Probe umfasst, wobei das Kit umfasst:
(a) Primer FOR und REV, wobei FOR und REV mit Sequenzen hybridisieren, die T flankieren;
(b) ein Plasmid, das eine Sequenz umfasst, die an FOR hybridisiert, gefolgt von einer Kontrollsequenz 1 (S1), gefolgt von einer Sequenz, die an REV hybridisiert ;
(c) ein Plasmid, das eine Sequenz umfasst, die an FOR hybridisiert, gefolgt von einer Kontrollsequenz 2 (S2), gefolgt von einer Sequenz, die an REV hybridisiert;
wobei T, S1 und S2 nicht identisch sind.

## Revendications

1. Méthode *in vitro* permettant de détecter la présence d'un acide nucléique comprenant une séquence cible (T) dans un échantillon, ladite méthode consistant :
a) À utiliser un échantillon comprenant potentiellement un acide nucléique comprenant T, T étant flanquée d'une séquence s'hybridant avec une amorce sens (FOR) et d'une séquence s'hybridant avec une amorce antisens (REV);
b) À transférer ledit échantillon dans un flacon V1;
c) À fournir un plasmide comprenant une séquence de contrôle 1 (S1), S1 étant flanquée d'une séquence s'hybridant avec FOR et d'une séquence s'hybridant avec REV; T et S1 n'étant pas identiques;
d) À transférer ledit plasmide de l'étape c) dans un flacon V2;
e) À fournir un plasmide comprenant une séquence de contrôle 2 (S2), S2 étant flanquée d'une séquence s'hybridant avec FOR et d'une séquence s'hybridant avec REV; T, S1 et S2 n'étant pas identiques;
f) À transférer ledit plasmide de l'étape e) dans lesdits flacons;
g) À extraire les acides nucléiques dans lesdits flacons;
h) À réaliser des réactions de PCR dans lesdits flacons à l'aide des amorces FOR et REV;
i) À séquencer les acides nucléiques amplifiés dans lesdits flacons;
la présence de T dans le flacon V1 indiquant la présence dudit acide nucléique comprenant T dans ledit échantillon si le séquençage dans le flacon V2 a occasionné la présence de S1 et S2, et si le séquençage dans le flacon V1 a occasionné la présence de T et S2.

2. Méthode selon la revendication 1, ledit échantillon étant un échantillon clinique.

3. Méthode selon la revendication 2, ledit échantillon clinique étant un échantillon de tissu ou un échantillon de liquide organique, de préférence d'un sujet humain.

4. Méthode selon la revendication 1, ledit acide nucléique qui comprend T étant un acide nucléique d'un microorganisme.

5. Méthode selon la revendication 3, ledit microorganisme étant choisi parmi le groupe comprenant les bactéries, les archéobactéries, les protozoaires, les champignons et les virus.

6. Méthode selon la revendication 1, ledit acide nucléique qui comprend T étant un oncogène, de préférence un oncogène humain.

7. Trousse de détection d'un acide nucléique comprenant une séquence cible (T) dans un échantillon, ladite trousse comprenant
(a) des amorces FOR et REV, FOR et REV s'hybridant avec les séquences flanquant T;
(b) un plasmide comprenant une séquence s'hybridant avec FOR, suivie d'une séquence de contrôle 1 (S1), suivie d'une séquence s'hybridant avec REV;
(c) un plasmide comprenant une séquence s'hybridant avec FOR, suivie d'une séquence de contrôle 2 (S2), suivie d'une séquence s'hybridant avec REV; T, S1 et S2 n'étant pas identiques.
